# EUROPEAN PATENT SPECIFICATION

(11) **EP 4 025 124 B1**
(45) Date of publication and mention of the grant of the patent: **15.05.2024**
(21) Application number: 20780773.6
(22) Date of filing: 04.09.2020
(51) Int. Cl.: A61B 5/08, A61B 5/087, A61B 5/00

(54) **A BREATH MONITORING APPARATUS FOR PRODUCING A CAPNOGRAM**
ATEMÜBERWACHUNGSGERÄT ZUR ERZEUGUNG EINES KAPNOGRAMMS
APPAREIL DE SURVEILLANCE DE RESPIRATION POUR PRODUIRE UN CAPNOGRAMME

(30) Priority: 05.09.2019 GB 201912772
(43) Date of publication of application: 13.07.2022
(73) Proprietor: Simpson, Keith, Newton Abbott, Devon TQ12 5NF (GB)
(72) Inventor: Simpson, Keith, Newton Abbott, Devon TQ12 5NF (GB)
(74) Representative: Greaves Brewster LLP
(86) International application number: PCT/GB2020/052118
(87) International publication number: WO 2021/044154

(56) References cited:
- US-A1- 2011 105 935
- US-A1- 2014 194 703
- US-A1- 2015 157 241
- US-A1- 2017 100 058
- US-A1- 2018 177 463

## Description

The present invention relates generally to a breath monitoring apparatus for producing a capnogram and a method of producing a capnogram for a patient, and finds particular, although not exclusive, utility in the field of capnography for humans and animals during periods when they may be anaesthetised.

Capnography includes devices for monitoring CO₂ levels in breath. The levels are typically shown in graph form. The graphs are produced in real-time (or with very slight delays of the order of a few seconds). These provide information to the anaesthetist or other clinician enabling them to provide better care to the patient and to prevent potential damage to the patient through inhaling CO₂ rich air.

Known devices are able to produce capnograms showing CO₂ levels over time, however there is no information as to which direction the air is moving, or if it is stationary. Accordingly, the patient may be breathing in CO₂ rich air which they have already breathed-out, or may have episodes of no breathing, neither of which can be determined from the capnogram.

US 2011/0105935 A1 describes a ventilation analysis system adapted to display a CO₂ waveform and breath flow dynamics signal.

US 2014/194703 A1 describes a method and system for obtaining a gas sample from a portion of a subject's breathing cycle.

In a first aspect, the present invention provides a breath monitoring apparatus for producing a capnogram for a patient, comprising airflow means arrangeable such that air flowing into and out of a patient during a breathing cycle of the patient are conducted through the airflow means; a CO₂ monitor for repeatedly determining the presence, and level, of CO₂in the air within the airflow means; an airflow direction sensor located within the airflow means, the airflow direction sensor for providing data distinguishing between periods when air is flowing into the patient, when air is flowing out of the patient, and when air in the airflow means is stationary; a processor for producing a graph on a display of an indication of CO₂ levels versus time, wherein the display includes different graphical qualities to distinguish between the level of CO₂ in the air flowing out of the patient, the level of CO₂ in the stationary air between breaths, and the level of CO₂ in the air flowing into the patient; characterised in that the processor is arranged to display the level of CO₂ as received from the CO₂ monitor a few seconds later than the airflow direction data..

In this manner, the clinician may visually be able to check the level of CO₂ associated with the patient's breathing in, breathing out and any pauses in breathing. The clinician may visually be able to check the degree of CO₂ rebreathing by the patient. The display provides information in an easily, and immediately, understandable format allowing for rapid response to potentially life-threatening situations.

The airflow means may include a tube or, a mask and a tube. The tube may be arranged to be insertable directly into the airway of the patient, such as an endotracheal tube. Alternatively, a mask may be used to cover the mouth and nostrils of the patient and a tube may lead from it, directing the breath.

The airflow direction sensor may include one or more of a pressure sensor, a temperature sensor, a micro-flow sensor, and an acoustic sensor. If a pressure sensor is employed it may detect airflow direction by means of a differential pressure sensor attached to two apertures provided in the airflow means tube arranged linearly with respect to one another such that one aperture is downstream of the other. The apertures may be connected to the sensor via tubes. Other means of determining airflow direction are contemplated.

The processor may be arranged to determine the airflow direction from signals sent by the airflow direction sensor. For example, if a differential pressure sensor is used, a higher pressure in one aperture compared to the other aperture may indicate breathing out and vice-versa may indicate breathing in. A balanced pressure across the two apertures may indicate no, or very little, breathing occurring.

The processor may be configured to display the level of CO₂ as received from the CO₂ monitor at a first sampling time, with the airflow direction data received from the airflow direction sensor from a second sampling time. The first sampling time may be the same as the second sampling time. In this regard the two may be only a few milliseconds apart yet still considered to be simultaneous. This may occur in a mainstream configuration where the CO₂ monitor and airflow direction sensor are both located within the same airflow means and located relatively close to one another. However, in a sidestream configuration, where the CO₂ monitor is located in a tube connected to, but not located directly in, the airflow means, the CO₂ level may relate to an airflow direction of an earlier period. In other words, there may be a delay between the two readings.

This may allow for the delay in the air reaching the CO₂ monitor to ensure that the two sets of data relate to the same air at the same time.

The delay between the two readings may be 10 milliseconds to 30 seconds.

The level of CO₂ may be expressed on the display as either or both of a percentage of CO₂ in the airflow, and a partial pressure of CO₂.

The display may include different colours to distinguish between the level of CO₂ in the air flowing out of the patient, the level of CO₂in the stationary air between breaths, and the level of CO₂in the air flowing into the patient. For instance, a white area under the graph line may be used to indicate breathing out, yellow may indicate no airflow, and blue may indicate breathing in. Other colours and combinations of colours are contemplated.

The airflow direction sensor and the CO₂ monitor may be combined in a single unit.

In a second aspect, the invention provides a method of producing a capnogram for a patient, comprising the steps of:
(a) providing a breath monitoring apparatus according to the first aspect;
(b) arranging the airflow means to conduct the airflow into and out of a patient during a breathing cycle;
(c) measuring the direction of airflow during the patient's breathing to distinguish between breathing in, breathing out, and no airflow;
(d) determining the presence, and level, of CO₂ in the air flowing in and out of the patient during the breathing cycle;
(e) producing a graph on a display showing an indication of CO₂ levels versus time, wherein the level of CO₂ is displayed as received from the CO₂ monitor a few seconds later than the airflow direction data;
(f) providing different graphical qualities to the graph to distinguish between the CO₂ as measured in the air flowing out of the patient, the CO₂ as measured in the stationary air between breaths, and the CO₂ as measured in the air flowing into the patient.

In this manner, the clinician may visually be able to check the level of CO₂ associated with the patient's breathing in, breathing out and any pauses in breathing. The clinician may visually be able to check the degree of CO₂ rebreathing by the patient. The display provides information in an easily, and immediately, understandable format allowing for rapid response to potentially life-threatening situations.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.
Figure 1 is a schematic diagram showing apparatus for producing capnograms; and
Figure 2 is an example capnogram.

The present invention will be described with respect to certain drawings but the invention is not limited thereto but only by the claims. The drawings described are only schematic and are non-limiting. Each drawing may not include all of the features of the invention and therefore should not necessarily be considered to be an embodiment of the invention. In the drawings, the size of some of the elements may be exaggerated and not drawn to scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that operation is capable in other sequences than described or illustrated herein. Likewise, method steps described or claimed in a particular sequence may be understood to operate in a different sequence.

Moreover, the terms top, bottom, over, under and the like in the description and the claims are used for descriptive purposes and not necessarily for describing relative positions. It is to be understood that the terms so used are interchangeable under appropriate circumstances and that operation is capable in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Similarly, it is to be noticed that the term "connected", used in the description, should not be interpreted as being restricted to direct connections only. Thus, the scope of the expression "a device A connected to a device B" should not be limited to devices or systems wherein an output of device A is directly connected to an input of device B. It means that there exists a path between an output of A and an input of B which may be a path including other devices or means. "Connected" may mean that two or more elements are either in direct physical or electrical contact, or that two or more elements are not in direct contact with each other but yet still co-operate or interact with each other. For instance, wireless connectivity is contemplated.

Reference throughout this specification to "an embodiment" or "an aspect" means that a particular feature, structure or characteristic described in connection with the embodiment or aspect is included in at least one embodiment or aspect of the present invention. Thus, appearances of the phrases "in one embodiment", "in an embodiment", or "in an aspect" in various places throughout this specification are not necessarily all referring to the same embodiment or aspect, but may refer to different embodiments or aspects. Furthermore, the particular features, structures or characteristics of any one embodiment or aspect of the invention may be combined in any suitable manner with any other particular feature, structure or characteristic of another embodiment or aspect of the invention, as would be apparent to one of ordinary skill in the art from this disclosure, in one or more embodiments or aspects.

Similarly, it should be appreciated that in the description various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Moreover, the description of any individual drawing or aspect should not necessarily be considered to be an embodiment of the invention. Rather, as the following claims reflect, inventive aspects lie in fewer than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some features included in other embodiments, combinations of features of different embodiments are meant to be within the scope of the invention, and form yet further embodiments, as will be understood by those skilled in the art. For example, in the following claims, any of the claimed embodiments can be used in any combination.

In the description provided herein, numerous specific details are set forth. However, it is understood that embodiments of the invention may be practised without these specific details. In other instances, well-known methods, structures and techniques have not been shown in detail in order not to obscure an understanding of this description.

In the discussion of the invention, unless stated to the contrary, the disclosure of alternative values for the upper or lower limit of the permitted range of a parameter, coupled with an indication that one of said values is more highly preferred than the other, is to be construed as an implied statement that each intermediate value of said parameter, lying between the more preferred and the less preferred of said alternatives, is itself preferred to said less preferred value and also to each value lying between said less preferred value and said intermediate value.

The use of the term "at least one" may mean only one in certain circumstances. The use of the term "any" may mean "all" and/or "each" in certain circumstances.

The principles of the invention will now be described by a detailed description of at least one drawing relating to exemplary features. It is clear that other arrangements can be configured according to the knowledge of persons skilled in the art without departing from the underlying concept or technical teaching, the invention being limited only by the terms of the appended claims.

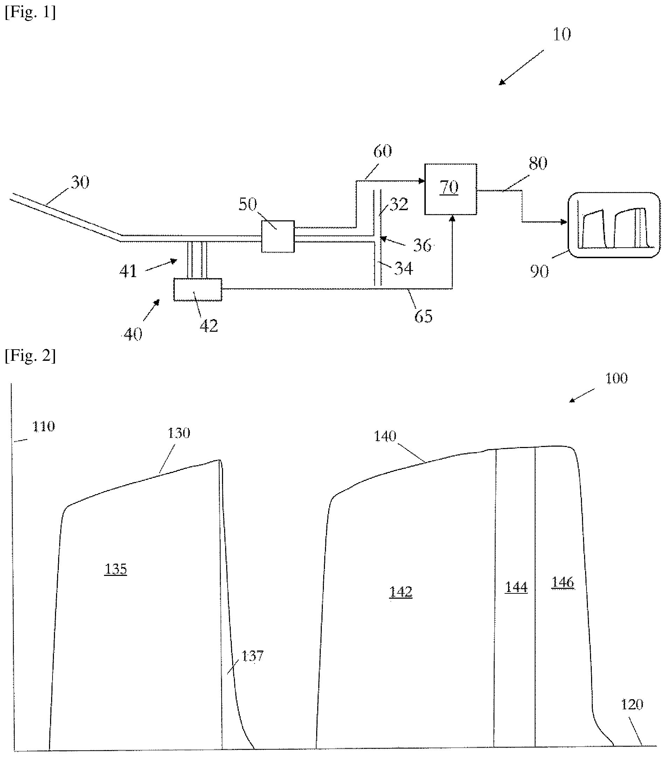

In Figure 1, a schematic diagram of apparatus 10 for producing a capnogram is shown. An endotracheal tube 30 is provided for placing within a patient's trachea. It leads to a T junction 36 where it diverges into two tubes 32, 34. One tube 32 allows air from an anaesthetic machine (not shown) to enter the tube 30. The other tube 34 allows breathed out air to be exhausted from the system. Valves may be arranged in the various tubes 30, 32, 34, to control the flow of air therethrough.

Air within the tube 30 may flow in either direction.

It is to be understood that instead of an endotracheal tube a mask (not shown) may be provided for placing over the patient's mouth and nose.

A CO₂ monitor 50, for repeatedly determining the level of CO₂ in the air within the tube 30, is arranged in that tube. The CO₂ levels are directed to a processor 70 via connection means 60.

The apparatus is shown in a "mainstream configuration" whereby the CO₂ monitor 50 is arranged in the tube 30 leading from the mask. It will be understood that in an alternative "sidestream configuration" (not shown) the CO₂ monitor 50 may be arranged in a tube leading off from the side of the tube 30 leading from the mask.

The airflow direction sensor 40 comprises two tubes 41, which may be fine-bore, leading off the endotracheal tube 30. These tubes 41 contain the same fluid (air) as the endotracheal tube 30. These tubes lead to a device 42 for determining the pressure difference in the fluid (air) within those two tubes. The result of that pressure difference is communicated to the processor 70 via connection means 65.

Although a mask 20 is shown it is to be understood that an endotracheal tube may be used in its place.

The processor 70 is arranged to determine the airflow direction within the tube 30 from the pressure difference communicated to it by the device 42. The processor then creates data signals for producing a capnogram based on the information received from the pressure sensor and CO₂ monitor. The capnogram is displayed on a VDU screen 90 which is connected to the processor 70 via connection means 80.

An example capnogram 100 is shown in Figure 2. The y-axis is percentage CO₂ in the monitored air. The x-axis is time.

A first graph 130 of CO₂ is shown on the left. The CO₂ levels rise relatively quickly, stay relatively constant for a time period and then reduce relatively quickly. On the VDU the area 135 under the graph will appear white in colour to indicate that the airflow direction is outwardly from the patient and that therefore the patient has breathed out air including this level of CO₂. However, the last portion 137 from the peak of CO₂ to when the CO₂ level is at a minimum will be coloured blue to indicate that the patient is breathing in. As can be seen, this portion is minimal and may be related to "rebreathing" expired CO₂ which is present in the bi-directional tube 30. The remainder of the time in which the patient is breathing in will be indicated by a blue line at zero percentage (not visible in the Figure) because there is no CO2 present.

A second graph 140 is shown on the right. Again, the CO₂ levels rise relatively quickly, stay relatively constant for a time period and then reduce relatively quickly. In a similar manner, the area 142 under the graph will appear white in colour to indicate that the airflow direction is outwardly from the patient and that therefore the patient has breathed out air including this level of CO₂. However, this white area only extends for approximately 60% of the total area under the graph 140.

An area 144 is shown immediately to the right of the white area 142. This will be coloured yellow on the VDU. This indicates that there is no movement of air. It is created by the patient's pause between breathing out and breathing in. The CO₂ level remains relatively constant as is shown by the relatively horizontal nature of the line of the graph 140 at the top of this yellow area 144. This yellow area 144 extends for approximately 20% of the total area under the graph 140.

Another area 146 is shown immediately to the right of the yellow area 144. This will be coloured blue on the VDU. This indicates that the direction of airflow is inwardly towards the patient and that therefore the patient has breathed in air including this level of CO₂. This blue area 146 extends for approximately 20% of the total area under the graph 140. The level of CO₂ falls relatively quickly as the patient continues breathing in with no CO₂ present.

Without the ability to determine the direction of airflow a clinician would only see the graph on the right as being of one colour and would not be able to determine if the CO₂ levels shown relate to breathing in, breathing out or the natural pause between the two.

By being able to determine the airflow direction and indicate it on the capnogram a clinician may be able to take steps to prevent the patient from breathing in air containing levels of CO₂ which may be harmful to the patient.

## Claims

1. A breath monitoring apparatus (10) for producing a capnogram for a patient, comprising airflow means (30) arrangeable such that air flowing into and out of a patient during a breathing cycle of the patient are conducted through the airflow means; a CO₂ monitor (50) for repeatedly determining the presence, and level, of CO₂in the air within the airflow means; an airflow direction sensor (40) located within the airflow means, the airflow direction sensor for providing data distinguishing between periods when air is flowing into the patient, when air is flowing out of the patient, and when air in the airflow means is stationary; a processor (70) for producing a graph on a display (90) of an indication of CO₂ levels versus time, wherein the display includes different graphical qualities to distinguish between the level of CO₂ in the air flowing out of the patient, the level of CO₂ in the stationary air between breaths, and the level of CO₂ in the air flowing into the patient; **characterised in that** the processor is arranged to display the level of CO₂ as received from the CO₂ monitor a few seconds later than the airflow direction data.

2. The breath monitoring apparatus of claim 1, wherein the airflow means (30) includes a tube or, a mask and a tube.

3. The breath monitoring apparatus of either one of claims 1 and 2, wherein the airflow direction sensor (40) includes one or more of a pressure sensor, a temperature sensor, a micro-flow sensor, and an acoustic sensor.

4. The breath monitoring apparatus of any preceding claim, wherein the processor (70) is arranged to determine the airflow direction from signals sent by the airflow direction sensor (40).

5. The breath monitoring apparatus of any preceding claim, wherein the level of CO₂ is expressed on the display (90) as either or both of a percentage of CO₂ in the airflow, and a partial pressure of CO₂.

6. The breath monitoring apparatus of any preceding claim, wherein the display (90) includes different colours to distinguish between the level of CO₂ in the air flowing out of the patient, the level of CO₂ in the stationary air between breaths, and the level of CO₂ in the air flowing into the patient.

7. The breath monitoring apparatus of any preceding claim, wherein the airflow direction sensor (40) and the CO₂ monitor (50) are combined in a single unit.

8. A method of producing a capnogram for a patient, comprising the steps of:
(a) providing a breath monitoring apparatus (10) according to any preceding claim;
(b) arranging the airflow means (30) to conduct the airflow into and out of a patient during a breathing cycle;
(c) measuring the direction of airflow during the patient's breathing to distinguish between breathing in, breathing out, and no airflow;
(d) determining the presence, and level, of CO₂ in the air flowing in and out of the patient during the breathing cycle;
(e) producing a graph on a display (90) showing an indication of CO₂ levels versus time, wherein the level of CO₂ is displayed as received from the CO₂ monitor (50) a few seconds later than the airflow direction data;
(f) providing different graphical qualities to the graph to distinguish between the CO₂as measured in the air flowing out of the patient, the CO₂as measured in the stationary air between breaths, and the CO₂ as measured in the air flowing into the patient.

## Patentansprüche

1. Atemüberwachungsgerät (10) zum Erzeugen eines Kapnogramms für einen Patienten, umfassend eine Luftströmungseinrichtung (30), die so anordenbar ist, dass Luft, die während eines Atemzyklus des Patienten in den Patienten hinein- und aus ihm herausströmt, durch die Luftströmungseinrichtung geleitet wird; einen CO₂-Monitor (50) zum wiederholten Bestimmen des Vorhandenseins von und des Gehalts an CO₂ in der Luft innerhalb der Luftströmungseinrichtung; einen Luftströmungsrichtungssensor (40), der sich innerhalb der Luftströmungseinrichtung befindet, wobei der Luftströmungsrichtungssensor dem Bereitstellen von Daten dient, die zwischen Zeiträumen unterscheiden, in denen Luft in den Patienten hineinströmt, in denen Luft aus dem Patienten herausströmt und in denen Luft in der Luftströmungseinrichtung stationär ist; einen Prozessor (70) zum Erzeugen eines Diagramms auf einer Anzeige (90) einer Angabe der CO₂-Gehalte über der Zeit, wobei die Anzeige verschiedene grafische Qualitäten enthält, um zwischen dem CO₂-Gehalt in der aus dem Patienten herausströmenden Luft, dem CO₂-Gehalt in der stationären Luft zwischen Atemzügen und dem CO₂-Gehalt in der in den Patienten hineinströmenden Luft zu unterscheiden; **dadurch gekennzeichnet, dass** der Prozessor so angeordnet ist, dass er den CO₂-Gehalt, wie er von dem CO₂-Monitor empfangen wird, einige Sekunden später als die Luftströmungsrichtungsdaten anzeigt.

2. Atemüberwachungsgerät nach Anspruch 1, wobei die Luftströmungseinrichtung (30) einen Schlauch oder eine Maske und einen Schlauch enthält.

3. Atemüberwachungsgerät nach einem der Ansprüche 1 und 2, wobei der Luftströmungsrichtungssensor (40) einen oder mehrere von einem Drucksensor, einem Temperatursensor, einem Mikroströmungssensor und einem akustischen Sensor enthält.

4. Atemüberwachungsgerät nach einem vorhergehenden Anspruch, wobei der Prozessor (70) so angeordnet ist, dass er die Luftströmungsrichtung aus Signalen, die durch den Luftströmungsrichtungssensor (40) gesendet werden, bestimmt.

5. Atemüberwachungsgerät nach einem vorhergehenden Anspruch, wobei der CO₂-Gehalt auf der Anzeige (90) entweder als ein Prozentsatz von CO₂ in der Luftströmung oder als CO₂-Partialdruck oder als beides ausgedrückt wird.

6. Atemüberwachungsgerät nach einem vorhergehenden Anspruch, wobei die Anzeige (90) verschiedene Farben enthält, um zwischen dem CO₂-Gehalt in der aus dem Patienten herausströmenden Luft, dem CO₂-Gehalt in der stationären Luft zwischen Atemzügen und dem CO₂-Gehalt in der in den Patienten hineinströmenden Luft zu unterscheiden.

7. Atemüberwachungsgerät nach einem vorhergehenden Anspruch, wobei der Luftströmungsrichtungssensor (40) und der CO₂-Monitor (50) in einer einzigen Einheit kombiniert sind.

8. Verfahren zum Erzeugen eines Kapnogramms für einen Patienten, umfassend die folgenden Schritte:
(a) Bereitstellen eines Atemüberwachungsgeräts (10) nach einem vorhergehenden Anspruch;
(b) Anordnen der Luftströmungseinrichtung (30), sodass sie die Luftströmung während eines Atemzyklus in den Patienten hinein- und aus ihm herausleitet;
(c) Messen der Richtung der Luftströmung während der Atmung des Patienten, um zwischen Einatmen, Ausatmen und keiner Luftströmung zu unterscheiden;
(d) Bestimmen des Vorhandenseins von und des Gehalts an CO₂ in der Luft, die während des Atemzyklus in den Patienten hinein- und aus ihm herausströmt;
(e) Erzeugen eines Diagramms auf einer Anzeige (90), die eine Angabe der CO₂-Gehalte über der Zeit zeigt, wobei der CO₂-Gehalt, wie er von dem CO₂-Monitor (50) empfangen wird, einige Sekunden später als die Luftströmungsrichtungsdaten angezeigt wird;
(f) Bereitstellen verschiedener grafischer Qualitäten für das Diagramm, um zwischen dem CO₂, wie es in der aus dem Patienten herausströmenden Luft gemessen wird, dem CO₂, wie es in der stationären Luft zwischen Atemzügen gemessen wird, und dem CO₂, wie es in der in den Patienten hineinströmenden Luft gemessen wird, zu unterscheiden.

## Revendications

1. Appareil de surveillance de respiration (10) destiné à produire un capnogramme pour un patient, comprenant un moyen d'écoulement d'air (30) pouvant être agencé de sorte que l'air s'écoulant dans et hors d'un patient pendant un cycle respiratoire du patient soit conduit à travers le moyen d'écoulement d'air ; un moniteur de CO₂ (50) destiné à déterminer de manière répétée la présence et le taux de CO₂ dans l'air à l'intérieur du moyen d'écoulement d'air ; un capteur de direction d'écoulement d'air (40) situé à l'intérieur du moyen d'écoulement d'air, le capteur de direction d'écoulement d'air servant à fournir des données faisant la distinction entre les périodes lorsque l'air s'écoule dans le patient, lorsque l'air s'écoule hors du patient et lorsque l'air dans le moyen d'écoulement d'air est stationnaire ; un processeur (70) destiné à produire un graphique sur un dispositif d'affichage (90) d'une indication des taux de CO₂ en fonction du temps, ledit dispositif d'affichage comprenant différentes qualités graphiques pour faire la distinction entre le niveau de CO₂ dans l'air sortant du patient, le taux de CO₂ dans l'air stationnaire entre les respirations et le taux de CO₂ dans l'air s'écoulant dans le patient ; **caractérisé en ce que** le processeur est agencé pour afficher le taux de CO₂ tel qu'il est reçu du moniteur de CO₂ quelques secondes après les données de direction d'écoulement d'air.

2. Appareil de surveillance de respiration selon la revendication 1, ledit moyen d'écoulement d'air (30) comprenant un tube, ou un masque et un tube.

3. Appareil de surveillance de respiration selon l'une quelconque des revendications 1 et 2, ledit capteur de direction d'écoulement d'air (40) comprenant un ou plusieurs capteurs parmi un capteur de pression, un capteur de température, un capteur de micro-débit et un capteur acoustique.

4. Appareil de surveillance de respiration selon l'une quelconque des revendications précédentes, ledit processeur (70) étant agencé pour déterminer la direction de l'écoulement d'air à partir des signaux envoyés par le capteur de direction d'écoulement d'air (40).

5. Appareil de surveillance de respiration selon l'une quelconque des revendications précédentes, ledit taux de CO₂ étant exprimé sur le dispositif d'affichage (90) sous la forme d'un pourcentage de CO₂ dans l'écoulement d'air ou d'une pression partielle de CO₂, ou les deux.

6. Appareil de surveillance de respiration selon l'une quelconque des revendications précédentes, ledit dispositif d'affichage (90) comprenant différentes couleurs pour faire la distinction entre le taux de CO₂ dans l'air s'écoulant hors du patient, le taux de CO₂ dans l'air stationnaire entre les respirations et le taux de CO₂ dans l'air s'écoulant dans le patient.

7. Appareil de surveillance de respiration selon l'une quelconque des revendications précédentes, ledit capteur de direction d'écoulement d'air (40) et ledit moniteur de CO₂ (50) étant combinés en une seule unité.

8. Procédé de production d'un capnogramme pour un patient, comprenant les étapes de :
(a) fourniture d'un appareil de surveillance de respiration (10) selon l'une quelconque des revendications précédentes ;
(b) agencement du moyen d'écoulement d'air (30) pour diriger l'écoulement d'air dans et hors d'un patient pendant un cycle respiratoire ;
(c) mesure de la direction de l'écoulement d'air pendant la respiration du patient pour faire la distinction entre l'inspiration, l'expiration et l'absence d'écoulement d'air ;
(d) détermination de la présence et du taux de CO₂ dans l'air s'écoulant dans et hors du patient pendant le cycle respiratoire ;
(e) production d'un graphique sur un dispositif d'affichage (90) montrant une indication des taux de CO₂ en fonction du temps, ledit taux de CO₂ étant affiché tel qu'il est reçu du moniteur de CO₂ (50) quelques secondes après les données de direction d'écoulement d'air ;
(f) fourniture de différentes qualités graphiques au graphique pour faire la distinction entre le CO₂ tel qu'il est mesuré dans l'air s'écoulant hors du patient, le CO₂ tel qu'il est mesuré dans l'air stationnaire entre les respirations, et le CO₂ tel qu'il est mesuré dans l'air s'écoulant dans le patient.
